# EUROPEAN PATENT SPECIFICATION

(11) **EP 0 548 188 B1**
(45) Date of publication and mention of the grant of the patent: **09.08.1995**
(21) Application number: 91916433.5
(22) Date of filing: 09.09.1991
(51) Int. Cl.: A61F 13/15

(54) **SHAPE AND ADHESIVE FASTENING MEANS FOR AN ABSORBENT ARTICLE**
FORM UND KLEBENDES BEFESTIGUNGSELEMENT FÜR SAUGFÄHIGE ARTIKEL
FORME ET MOYENS DE FIXATION ADHESIVE D'UN ARTICLE ABSORBANT

(30) Priority: 12.09.1990 US 580985
(43) Date of publication of application: 30.06.1993
(73) Proprietor: THE PROCTER & GAMBLE COMPANY, Cincinnati, Ohio 45202 (US)
(72) Inventor: PAPA, Alyce, Johnson, Wyoming, OH 45215 (US); OSBORN, Thomas, Ward, III, Cincinnati, OH 45235 (US); GLACKIN, Allison, Haack, Wyoming, OH 45215 (US); AMOS, Charles, William, Jr., Cincinnati, OH 45231 (US); RILEY, Raphael, Joseph, Cincinnati, OH 45240 (US)
(74) Representative: Hirsch, Uwe Thomas
(86) International application number: US9106400
(87) International publication number: WO9204000

(56) References cited:
- EP-A- 0 211 376
- EP-A- 0 248 584
- EP-A- 0 335 252
- DE-U- 8 609 587
- GB-A- 2 165 158
- GB-A- 2 171 915
- GB-A- 2 195 541
- US-A- 4 608 047
- US-A- 4 950 264

## Description

The present invention is directed to a relatively thin, absorbent article of an improved shape, such as a sanitary napkin, and to an improved adhesive fastening means for attaching such an absorbent article to an undergarment.

### BACKGROUND OF THE INVENTION

This invention is concerned with absorbent articles such as sanitary napkins, pantiliners, and incontinent pads that are designed to absorb and retain liquid and other discharges from the human body and to prevent body and clothing soiling. The present invention is particularly concerned with absorbent articles such as sanitary napkins that are relatively thin and flexible, and with adhesive fastening means for attaching such an absorbent article to an undergarment.

Sanitary napkins of a wide variety of shapes and dimensions are currently used by women for the collection of menses and other bodily discharges.

The tendency has been to develop sanitary napkins which are increasingly thinner, and thus more comfortable and less obtrusive than prior sanitary napkins. Recently, efforts have also been directed at developing thin sanitary napkins which have the capacity to absorb and contain medium to high menstrual discharges. Previously, such discharges could only be handled by relatively thick sanitary napkins. An example of a thin sanitary napkin having a capacity great enough to handle medium to high menstrual flows is disclosed in U.S. Patent 4,950,264, issued to Osborn, III, on August 21, 1990 which is incorporated by reference herein.

Efforts have also been directed at providing sanitary napkins which conform closely to the shape of the female urogenital and buttocks region. In addition to serving as an example of a thin sanitary napkin, the sanitary napkin disclosed in the above-mentioned Osborn patent also serves as an example of such a anatomically-conforming sanitary napkin. Another example of an anatomically-conforming sanitary napkin is disclosed in EP-A-0 335 252 entitled "Absorbent Article" in the name of Kenneth Barclay Buell, although the disclosure of the Buell patent application is not limited to thin sanitary napkins.

The sanitary napkins currently in use employ different means by which they may be attached to the wearer's undergarments. The present invention is directed to adhesive means for attaching sanitary napkins to the wearer's undergarments, or panties. (These adhesive fastening means may also be referred to herein as "panty adhesives" or "panty fastening adhesives"). In particular, the present invention is directed to solving some of the problems that are particularly prevalent in thin sanitary napkins. Such problems include the tendency of the lateral edges of thin sanitary napkins to roll over and bunch, and the tendency of the end edges to flip over and bring the panty adhesives in contact with the wearer's genetalia.

As a result of the foregoing, a need exists for a thin, absorbent article, such as a sanitary napkin, of an improved shape that will be less subject to the above-described problems. A need also exists for an improved adhesive fastening means which overcomes the drawbacks inherent in the adhesive fastening means currently in use and which will also allow such thin sanitary napkins to conform to the wearer's body.

Therefore, it is an object of the present invention to provide a thin absorbent article, such as a sanitary napkin, having a shape that will be less subject to edge and end roll over and bunching problems.

It is also an object of the present invention to provide an adhesive fastening means which reduces the prior problems of bunching, rolling over, and side and end flipping associated with prior thin absorbent articles.

It is another object of the present invention to provide a adhesive fastening means which will allow thin absorbent articles to conform to the wearer's body.

These and other objects of the present invention will be more readily apparent when considered in reference to the following description and when taken in conjunction with the accompanying drawings.

### SUMMARY OF THE INVENTION

The present invention provides a thin, absorbent, anotomically-conforming absorbent article, such as a sanitary napkin, and an improved adhesive fastening means for attaching such an absorbent article to an undergarment. The absorbent article of the present invention has two surfaces, a body surface, and a garment surface. The absorbent article has a central region disposed between two end regions, and a longitudinal centerline and a transverse centerline. The absorbent article of the present invention also has two spaced apart longitudinal edges, two spaced apart ends, and four corners, which together form the periphery of the absorbent article. In a preferred embodiment, the longitudinal edges of the absorbent article are inwardly arcuate with relation to each other in the central region of the absorbent article and convex outwardly in the end regions. The end edges of the absorbent article are also convex outwardly. The absorbent article generally has a caliper of less than or equal to about 5 millimeters. The absorbent article of the present invention comprises a liquid pervious topsheet; a liquid resistant backsheet which has a core-facing side and a garment side; an absorbent core positioned between the topsheet and the backsheet; and an adhesive fastening means on the garment-facing side of the backsheet for attaching the absorbent article to a garment.

The fastening means comprises at least one zone of adhesive having outside edges and ends spaced a distance of 6 millimetres, plus or minus 3 millimetres, from the portion of the periphery which bounds the end regions of the absorbent article.

In several of the preferred embodiments, the fastening means comprises two spaced apart longitudinally-oriented zones of adhesive, which are preferably strips of adhesive, that are disposed on opposite sides of the longitudinal centerline of the absorbent article. The strips each have a longitudinal dimension, a transverse dimension, and ends. The strips have an inside longitudinal edge and an outside longitudinal edge. The outside longitudinal edge of each strip is disposed further away from the longitudinal centerline of the absorbent article than the inside edge of the strip. In the preferred embodiments in which the fastening means comprises two spaced apart strips of adhesive, the distance between the inside edges of the strips is between about 8 millimeters and about 30 millimeters in the central region of the absorbent article. In a first preferred embodiment of the present invention, the inside edge and the outside edge of the same strip are essentially parallel. In a second preferred embodiment, the distance between the outside edges of each of the strips and the periphery of the absorbent article is approximately the same along the longitudinal edges of the absorbent article. In the first and second preferred embodiments, the distance between the outside edges of the strips is greater than about 47 millimeters in the central region of the absorbent article. In a third preferred embodiment the strips are generally arcuate. In the third embodiment, the strips are spaced further apart in the end regions of the absorbent article than they are in the central region. In the third embodiment, the distance between the outside edges of the strips is greater than about 35 mm in the central region of the absorbent article. In the end regions, the distance between the outside edges of the strips is greater than about 47 mm and less than or equal to about 90 mm. In a fourth embodiment, the strips are also generally arcuate and are spaced further apart in the end regions of the absorbent article than they are in the central region. In the fourth embodiment, the strips may be inwardly arcuate with relation to each other in the central region of the absorbent article and outwardly arcuate with relation to each other in at least one of the end regions of the absorbent article.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is to be taken in conjunction with the accompanying drawings in which like designations are used to designate substantially identical elements, and in which:
FIG. 1 is a bottom plan view of a sanitary napkin of a preferred configuration which shows the improved panty fastening adhesive of the present invention.
FIG. 1A is a bottom plan view of a sanitary napkin having an alternative adhesive configuration in which the zones of adhesive comprise multiple parallel strips or lines of adhesive.
FIG. 2 is a schematic cross-sectional view of the preferred sanitary napkin shown in FIG. 1 taken along line 2-2 of FIG. 1.
FIG. 3 is a bottom plan view of a sanitary napkin having an alternative adhesive configuration.
FIG. 3A is a bottom plan view of a sanitary napkin having an alternative adhesive configuration in which there is only one zone of adhesive.
FIG. 4 is a bottom plan view of a sanitary napkin having another alternative adhesive configuration.
FIG. 5 is a bottom plan view of a sanitary napkin having another alternative adhesive configuration.
FIG. 5A is a bottom plan view of a sanitary napkin having another alternative adhesive configuration in which the edges of the strips are at least partially comprised of discrete rectilinear segments.
FIG. 6 is a bottom plan view of a sanitary napkin having another alternative adhesive configuration in which there are longitudinally-oriented strips disposed on opposite sides of the longitudinal centerline of the sanitary napkin, and zones of adhesive positioned near the corners of the sanitary napkin.

### DETAILED DESCRIPTION OF THE INVENTION

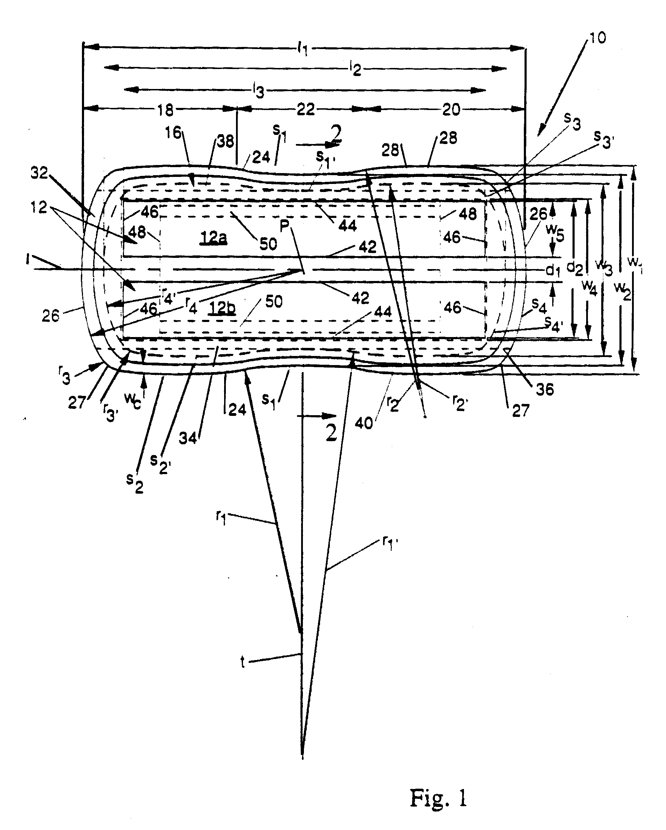

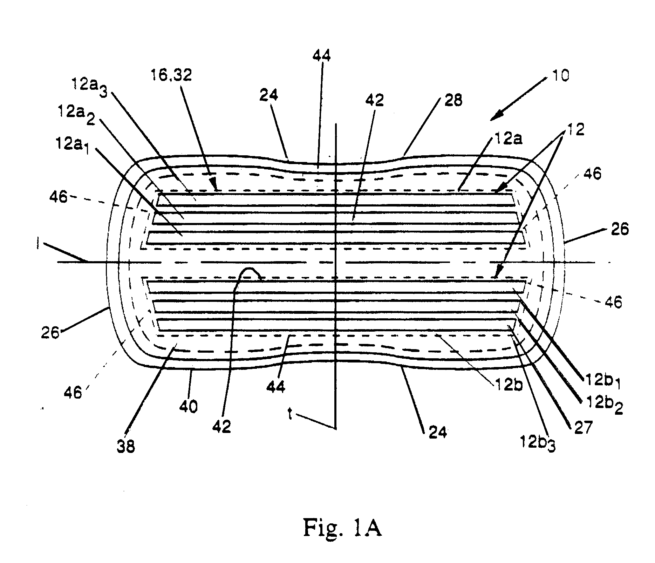

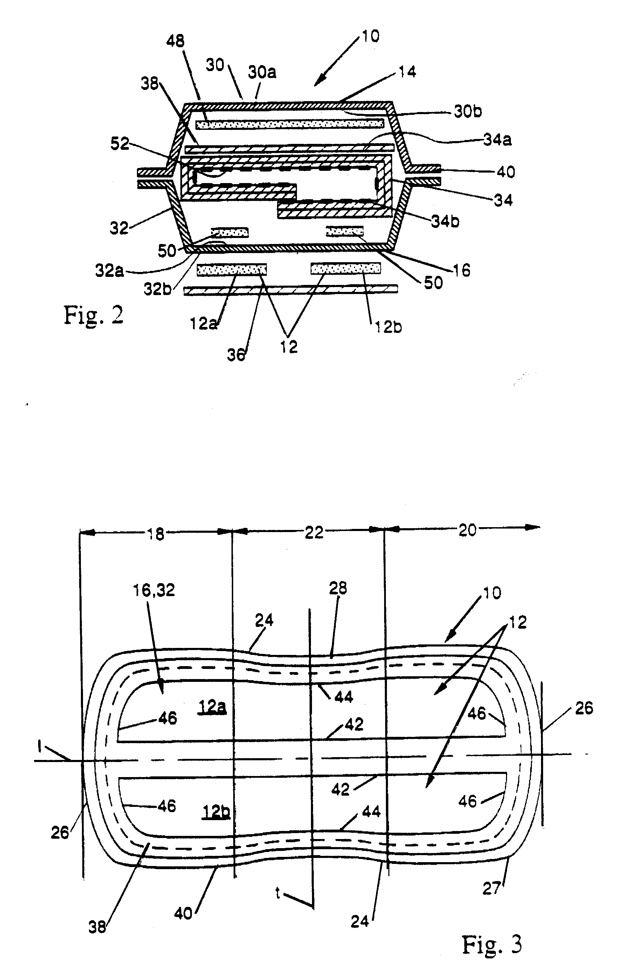

Referring now to the drawings, and more particularly to FIGS. 1 and 2, there is shown a preferred embodiment of a disposable absorbent article of the present invention, sanitary napkin 10. As used herein, the term "absorbent article" refers to articles which absorb and contain body exudates and more specifically refers to articles which are placed against or in proximity to the body of the wearer to absorb and contain the various exudates discharged from the body. The term "absorbent article" is intended to include sanitary napkins, pantiliners, and incontinent pads (articles worn in the crotch region of a garment). The term "disposable" refers to articles which are intended to be discarded after a single use (that is, articles which are not intended to be laundered or otherwise restored or reused). The sanitary napkin 10 illustrated in FIG. 1 includes the improved panty fastening adhesive 12 of the present invention. The panty fastening adhesive 12 of the present invention may also be referred to in the patents incorporated by reference herein as the "adhesive attachment means".

The sanitary napkin 10 is shown in FIG. 1 as viewed from its garment surface 16. The garment surface 16 is intended to be placed adjacent to the wearer's undergarments when the sanitary napkin 10 is worn. The sanitary napkin 10 has a body surface 14 (shown in FIG. 2) on the opposite side that is intended to be worn adjacent to the body of the wearer. As shown in FIG. 1, the sanitary napkin 10 of the present invention has two end regions, which are designated as first end region 18 and second end region 20. A central region 22 is disposed between the end regions 18 and 20. The sanitary napkin 10 has a longitudinal centerline l and a transverse centerline t that intersect at a point p in the center of the sanitary napkin 10. FIG. 1 shows that the sanitary napkin 10 has two spaced apart longitudinal edges 24, two spaced apart end edges (or "ends") 26, and four corners 27, which together form the periphery 28 of the sanitary napkin 10. (It should be understood that the longitudinal edges 24 and the ends 26 may be considered to each comprise a portion of the corners 27, and, unless specified, the corners 27 may not be referred to as separate components of the sanitary napkin 10.)

As shown in FIG. 2, the sanitary napkin 10 of the present invention comprises a liquid pervious topsheet 30, a liquid resistant backsheet (or "barrier means") 32, and an absorbent core 34 positioned between the topsheet 30 and the backsheet 32. The topsheet 30 permits liquids to readily transfer through its thickness toward the absorbent core 34. The topsheet 30 has a body-facing side (or "body surface") 30a and a core-facing side 30b. The body-facing side 30a of the topsheet generally comprises the body surface 14 of the sanitary napkin 10. The backsheet 32 prevents the exudates absorbed and contained in the absorbent core 34 from wetting articles which contact the sanitary napkin 10 such as the wearer's panties. The backsheet 32 has a core-facing side 32a and a garment side 32b. The garment side 32b of the backsheet generally comprises the garment surface 16 of the sanitary napkin 10. The absorbent core (or simply "the core") 34 serves as a means of absorbing bodily fluids. The absorbent core 34 has a body-facing side (or "first major surface") 34a and a garment-facing side (or "second major surface") 34b. As shown in FIGS. 1 and 2, the adhesive fastening means 12 of the present invention is disposed on the garment side 32b of the backsheet 32.

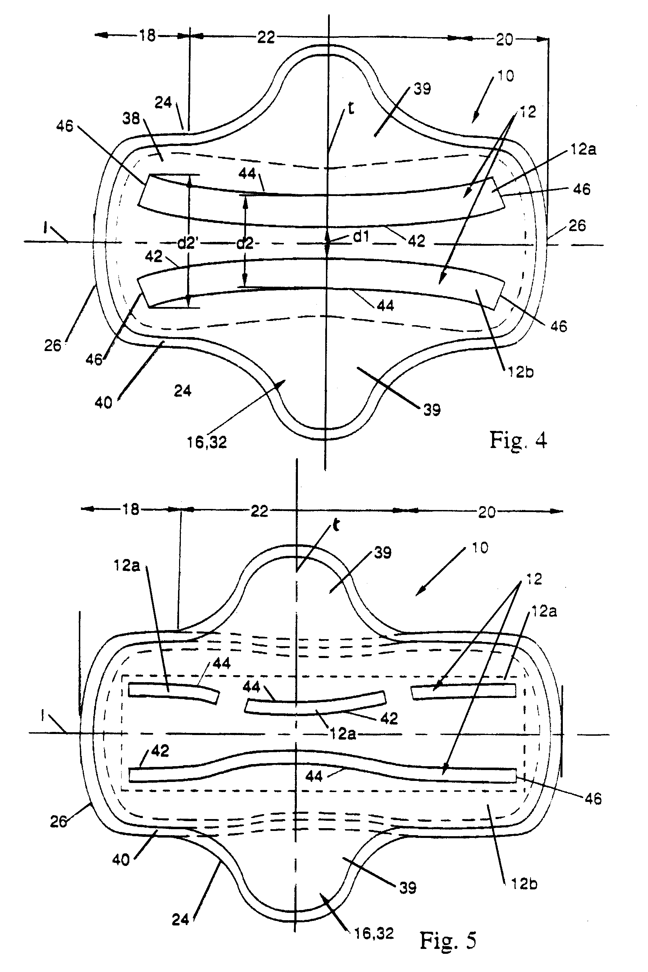

The sanitary napkin 10 of the present invention can be provided with any optional additional elements that are known in the art, including a removable release liner 36. The removable release liner 36 covers the adhesive fastening means 12 in order to keep the adhesive from drying out or sticking to a surface other than the crotch portion of the garment prior to use. The sanitary napkin 10 of the present invention can also be provided with wicking layers disposed between the absorbent core 34 and either the topsheet 30, the backsheet 32 or both. As shown in FIG. 2, the sanitary napkin 10 of the present invention is preferably provided with a wicking layer between the topsheet 30 and the absorbent core 34. This wicking layer is referred to as a secondary topsheet (or "wipe acquisition sheet") and is designated by reference number 38. In addition, the sanitary napkin 10 of the present invention may be provided with side flaps or "wings" 39 such as those provided on the sanitary napkin shown in FIG. 4.

The particular characteristics of the component parts or elements of the sanitary napkin 10 of the present invention (other than the preferred shapes and dimensions of those elements, which are described herein) are set forth in greater detail in the patents previously incorporated by reference herein. A detailed description of the central region and the two end regions is set forth in U.S. Patent 4,690,680 issued to Higgins on September 1, 1987, which is hereby also incorporated by reference herein. A detailed description of the characteristics of the topsheet 30, backsheet 32, and absorbent core 34, and the optional wipe acquisition sheet 38 is contained in U.S. Patent 4,950,264, issued to Osborn on August 21, 1990. (It should be noted that the topsheet 30, wipe acquisition sheet 38, (an optional wet-laid tissue sheet), and the absorbent core 34 discussed above are referred to as an "absorbent means" in the Osborn patent.)

The improved panty fastening adhesive 12 of the present invention is suitable for use with sanitary napkins having a flexure-resistance of any of the values disclosed in the Osborn patent. The flexure-resistance of the sanitary napkin 10 of the present invention can be as great as about 700 grams or more as measured using the modified Circular Bend Procedure described in the Osborn patent (without, of course, any optional removable release liner 36 attached to the sanitary napkin). The caliper of the sanitary napkin 10 of the present invention can also be any of the values disclosed in the Osborn patent. Preferably, however, the caliper should be less than or equal to about 5 mm measured according to the test set forth in the Osborn patent, again without any optional release liner attached during the testing.

The component parts of the sanitary napkin 10 of the present invention are secured together in the following manner. As shown in FIG. 2, the secondary topsheet 38 is preferably attached to the core-facing side 30b of the topsheet 30 by a first attachment means designated 48. As shown in FIG. 1, the first attachment means 48 preferably comprises an adhesive. The adhesive has transverse edges which appear in FIG. 1 to be generally perpendicular to the ends of a second attachment means 50. The second attachment means or "core attachment means" 50 serves to attach the absorbent core 34 to the core-facing side 32a of the backsheet 32. The core attachment means 50 preferably comprises two spaced apart longitudinally-oriented strips of adhesive disposed on opposite sides of the longitudinal centerline l. In the preferred embodiment shown in FIG. 2, the absorbent core 34 comprises a laminate of a layer of superabsorbent polymer material and an air-laid tissue. The third attachment means 52 serves to bond together the layers of the laminate. It should be understood that FIG. 2 is a simplified schematic representation of the cross-section of the preferred sanitary napkin 10 shown in FIG. 1. In FIG. 2, some of the components of the sanitary napkin 10 are shown as being spaced away from adjacent components for purposes of illustration. It should be understood, however, that in the preferred embodiment of the present invention, those components which are connected by the above-described attachment means will actually be secured together.

The shape of the sanitary napkins that utilize the improved adhesive fastening means 12 of the present invention can vary. For instance, the adhesive fastening means 12 can be used with the sanitary napkins disclosed in the aforementioned U.S. Patent 4,950,264, issued to Osborn on August 21, 1990. The adhesive fastening means 12 can, however, also be used with sanitary napkins of other configurations. Preferably, the shape of the sanitary napkin on which the adhesive fastening means 12 is placed is one that tends to reduce the aforementioned roll over and bunching problems. A preferred embodiment of such a sanitary is shown in FIG. 1.

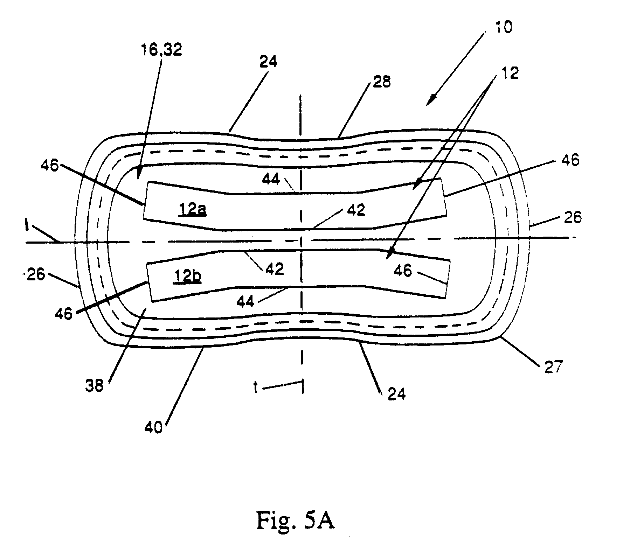

The sanitary napkin 10 shown in FIG. 1 has longitudinal edges 24 which are inwardly arcuate with relation to each other in the central region 22 and convex outwardly (i.e., outwardly arcuate with relation to each other) in the end regions 18 and 20. As shown in FIG. 1, the end edges 26 of this preferred sanitary napkin 10 are also convex outwardly (or outwardly arcuate with relation to each other). Preferably, both the direction of curvature and the radii of curvature of the edges 24 and 26 of the sanitary napkin 10 are established to optimize fit and, to the extent possible, reduce edge roll over problems. As shown in FIG. 1, this is done by increasing the radii of curvature or "squaring off" the longitudinal edges 24 in the end regions 18 and 20 of the sanitary napkin 10 and also squaring off the end edges 26 of the sanitary napkin 10. (The aforementioned edges are squared off in relation to prior sanitary napkins with rounded longitudinal and end edges.) The preferred sanitary napkin 10 shown in FIG. 1 is symmetrical about both the longitudinal and transverse centerlines, however, such symmetry is not absolutely necessary. For instance, as shown in FIG. 5, the sanitary napkin 10 of the present invention can be symmetrical about the longitudinal centerline l and asymmetrical about the transverse centerline. Preferably, the sanitary napkin 10 of the present invention will be at least symmetrical about the longitudinal centerline.

The dimensions of this preferred sanitary napkin 10 can also vary. The dimensions of the sanitary napkin 10 should be large enough to accomodate the adhesive fastening means 12 that is described in greater detail below. The dimensions should not be so great relative to the dimensions of the adhesive fastening means 12, however, that portions of the sanitary napkin 10 extend an inordinate distance beyond the edges of the adhesive fastening means 12. If the longitudinal edges and end edges of the sanitary napkin are unfastened and extend beyond the edges of the adhesive fastening means 12 an inordinate distance, they will cause the above-described roll over problems to be accentuated. In the preferred embodiment of the sanitary napkin 10 shown in FIG. 1, the overall length l₁ of the sanitary napkin 10 is about 227 millimeters (mm) measured along the longitudinal centerline l. In other embodiments, the length of the sanitary napkin 10 (as well as the other dimensions provided below) could be more or less than the amounts specified herein so long as the objectives set forth in the preceding paragraph are achieved. The overall width w₁ of the sanitary napkin 10 at the widest portion (in the end regions 18 and 20) is about 103 mm measured in a direction generally parallel to the transverse centerline t. The overall width w₂ of the sanitary napkin 10 at the narrowest portion (in the central region 22) is about 83 mm measured along the transverse centerline t. As shown in FIG. 1, if the topsheet 30 and the backsheet 32 are bonded by a heat/pressure process, there will also be a "crimped" area 40 around at least a portion of the periphery 28 of the sanitary napkin 10. Preferably, the width w_{c} of the crimped area 40 is about 6 mm.

The periphery 28 of the sanitary napkin 10 shown in FIG. 1 is formed by a number of curved segments or lines. The inwardly arcuate segments designated s₁ form those portions of the longitudinal edges 24 in the central region 22 of the sanitary napkin 10. The convex outwardly segments designated s₂ form the portions of the longitudinal edges 24 of the sanitary napkin 10 in the end regions 18 and 20. The segments designated s₃ form the corners 27 of the sanitary napkin 10. The convex outwardly segments designated s₄ form the end edges 26 of the sanitary napkin 10. The ends of the segments described above are located at those places where the direction or radius of curvature of the segments change. There are preferred radii of curvature for these segments. The inwardly arcuate segments s₁ in the central region 22 of the sanitary napkin 10 have a center located on the transverse centerline and a radius of curvature r₁ of between about 100-300 mm, and preferably is about 127 mm. The convex outwardly segments s₂ that form the longitudinal edges 24 in the end regions 18 and 20 of the sanitary napkin 10 have a center that is located about 94 mm from the longitudinal centerline and about 73 mm from the transverse centerline. The radius of curvature r₂ of the convex outwardly segments s₂ is less than or equal to about 500 mm, and preferably is about 146 mm. The radius of curvature r₃ of the segments s₃ that form the corners 27 of the sanitary napkin 10 is between about 10-30 mm, and preferably is about 17 mm. The convex outwardly segments s₄ that form the end edges 26 of the sanitary napkin 10 have a center which lies at the intersection of the longitudinal and transverse axes. The radius of curvature r₄ of the segments s₄ that form the end edges 26 should be between about 50-200 mm, and preferably is between about 110-200 mm, and most preferably is about 113 mm. The above radii of curvature should, of course, be selected so that each segment will align with the adjacent segments.

As shown in FIG. 1, the secondary topsheet 38 preferably has the same general shape as the overall shape of the sanitary napkin 10. The dimensions of the secondary topsheet 38 include a length l₂ of about 205 millimeters (mm) measured along the longitudinal centerline l. The overall width w₃ of the secondary topsheet 38 at the widest portion (in the end regions 18 and 20) is about 81 mm measured in a direction generally parallel to the transverse centerline t. The overall width w₄ of the secondary topsheet 38 at the narrowest portion (in the central region 22) is about 65 mm measured along the transverse centerline t.

The periphery 28' of the secondary topsheet 38 is formed by segments which are designated s₁', s₂', s₃', and s₄'. The radii of curvature of the segments s₁', s₂', s₃', and s₄' are also designated by a prime symbol in FIG. 1. The radii of curvature of the respective segments s₁', s₂', s₃', and s₄' are as follows: r₁' about 204 mm; r₂' about 135 mm; r₃' about 6 mm; and, r₄' about 102 mm.

The absorbent core 34 of the sanitary napkin 10 of the present invention (as described above) is preferably a laminate. The laminate is preferably in an "e"-folded configuration when viewed in transverse cross-section (as in FIG. 2) and rectangular in plan view as shown in FIG. 1. In the preferred embodiment shown, the length of the absorbent core 34 is preferably about 191 mm, and the width is about 65 mm.

The characteristics of the adhesive fastening means (or "fastening means") 12 of the present invention will now be discussed. The adhesive fastening means 12 of the present invention should be of such a configuration that it reduces or eliminates the aforementioned edge and end roll over and bunching problems. The adhesive fastening means 12 should generally maintain at least portions of the sanitary napkin 10 along the longitudinal edges 24 of the sanitary napkin 10 in constant contact with the undergarments of the wearer. The adhesive fastening means 12 should also allow the sanitary napkin 10 to assume a shape that will conform to the body of the wearer. Preferably, the adhesive fastening means 12 will allow the sanitary napkin 10 to deform into a W-shape similar to that shown in FIG. 11 of the aforementioned Buell patent application. In addition, the adhesive fastening means 12 should preferably also assume a configuration that will fit at least partially into the wearer's gluteal groove similar to that of the sanitary napkin shown in FIG. 13 of the Buell patent application. (Although those particular figures of the Buell application are not limited to thin sanitary napkins.)

It has been found that it is desirable that the overall width of the adhesive fastening means 12 should be as close as possible to the total width of the crotch region of the wearer's panties. In addition, it has been found that it is preferably that there be an area along the longitudinal centerline l of the sanitary napkin 10 where the backsheet 32 is unattached to the wearer's undergarments to avoid interference with the body-conforming characteristics of the sanitary napkins described herein. The portion of the backsheet 32 which is unattached to the wearer's undergarments can, therefore, separate from the wearer's undergarments. While not wishing to be bound by any particular theory, it is believed that the separation of a portion of the backsheet 32 from the undergarments along the longitudinal centerline of the sanitary napkin 10 will facilitate the ability of the sanitary napkin 10 to form the desired W-shape described above. It is believed that the separation will allow those parts of the entire sanitary napkin 10 that are adjacent the longitudinal centerline to move and thus help to create the hump-like characteristic that forms the middle of the desired W-shape cross-sectional profile.

In the embodiment shown in FIG. 1, the adhesive fastening means 12 comprises two spaced apart longitudinally-oriented zones of adhesive, such as strips of adhesive 12a and 12b, that are disposed on opposite sides of the longitudinal centerline l of the sanitary napkin 10. The term "longitudinally-oriented", as used herein refers to zones which have a dimension that runs in generally the same direction as the longitudinal centerline l that is longer than the dimension of the zone that runs in generally the same direction as the transverse centerline t. It should be understood that each zone of adhesive can be comprised of multiple parallel strips or lines of adhesive as shown in FIG. 1A, or adhesive arranged in any other configuration within the zones 12a and 12b. In addition, the zones or strips 12a and 12b can be either continuous or intermittent. The materials which comprise the strips 12a and 12b can be any of the materials described in U.S. Patent 4,690,680 issued to Higgins, which is incorporated herein. The strips 12a and 12b can be of any shape when viewed from the bottom (as in FIG. 1). In the particular preferred embodiment shown in FIG. 1, however, the strips 12a and 12b are preferably each generally rectangular in shape. In FIG. 1, the strips 12a and 12b are also parallel to the longitudinal centerline. Preferably, the strips 12a and 12b are of the same shape and dimensions. In addition, the strips 12a and 12b are preferably symmetrically disposed about the longitudinal centerline l of the sanitary napkin 10.

Each of the strips 12a and 12b has two longitudinal edges (referred to generally as "edges"), an inside longitudinal edge (or "inside edge") 42, an outside longitudinal edge (or "outside edge") 44, and two ends 46. As shown in FIG. 1, the outside longitudinal edges 44 of each strip 12a and 12b is disposed further away from the longitudinal centerline l of the sanitary napkin 10 than its inside longitudinal edge 42. In addition, each strip 12a or 12b has a length, or longitudinal dimension, l₃, and a width, or transverse dimension, w₅. When the strips 12a and 12b are rectangularly-shaped, the longitudinal and transverse dimensions of the strips 12a and 12b are constant across the width and along the length of the strips 12a and 12b, respectively. It is also within the scope of the present invention that the strips may be wider in some places than in other places. Likewise, the length of a strip as measured across its width may differ (for instance, if the ends of the strips curved).

In the preferred embodiment of the present invention shown in FIG. 1, the exact dimensions of the adhesive fastening means 12 are as follows. The distance d₁ between the inside edges 42 of the strips 12a and 12b is between about 8 mm and about 30 mm (or more preferably, between about 8 mm and about 15mm). Still more preferably, the distance d₁ between the inside edges 42 is between about 10 mm and about 12 mm. Most preferably, the distance d₁ between the inside edges 42 is about 11 mm. The distance d₁ between the inside edges 42 should be within the above ranges at least in the central region 22 of the sanitary napkin 10. The distance between the inside edges 42 of the strips, d₁, can be the same along the entire length of the strips as in the case of strips 12a and 12b in FIG. 1. Alternatively, the distance between the inside edges 42 of the strips can vary so that the distance between the inside edges 42 of the strips is within the above ranges in the central region 22 of the sanitary napkin 10 and the distance between the inside edges 42 is different in the end regions 18 and 20. In the embodiment shown in FIG. 1, the distance d₂ between the outside edges 44 of the strips 12a and 12b (that is, the "overall width" of the adhesive fastening means 12) should be greater than about 47 mm in the central region 22. Preferably, the distance d₂ between the outside edges 44 is between about 55 mm and about 70 mm in the central region 22. Most preferably, the distance d₂ between the outside edges 44 is about 60 mm in the central region 22. The distance between the outside edges 44 of the strips 12a and 12b should preferably be at least 6 mm from the periphery 28 of the sanitary napkin 10.

It is believed that a spacing of approximately 60 mm between the outside edges 44 of the strips in the central region will result in the overall width of adhesive fastening means 12 being approximately the same as the width of the crotch portion of a majority of panties currently worn. While not wishing to be bound by any particular theory, it is believed that if the overall width of the adhesive fastening means 12 corresponds with the width of the crotch portion of the wearer's panties, side flipping will be reduced. The reduction in side flipping is believed to be attributable to the fact such a spacing of the strips will tend to hold the sanitary napkin 10 in place at the widest portion of the crotch of the panties. Such a spacing is believed to be helpful because side flipping is believed to be at least partially due to the forces exterted on the sanitary napkin by the outside edges of the panties, particularly by the panty leg elastics.

The length of the strips 12a and 12b preferably should be as close to the overall length of the sanitary napkin 10 as possible. This will hold the end edges 26 of the sanitary napkin 10 to the wearer's undergarments, and thereby reduce the end flipping problem described above. The ends 46 of the strips 12a and 12b should be no more than about 30 mm from each end edge 26 of the sanitary napkin 10. Preferably, the ends 46 should be no more than about 20 mm away from the end edges 26, more preferably no more than about 10 mm, and most preferably no more than about 6 mm. The ends 46 of the strips 12a and 12b should, however, not be too close to the end edges 26 of the sanitary napkin 10. If the ends 46 of the strips 12a and 12b come too close to the end edges 26 of the sanitary napkin 10, the slightest amount of end flipping will cause the adhesive to come in contact with the wearer's body. The minimum distance the ends 46 of the strips should be from the end edges 26 of the sanitary napkin is at least about 6 mm plus or minus about 3 mm. (All measurements are to the point on the periphery 28 of the sanitary napkin 10 closest to the ends of the strips.) In the preferred embodiment of the present invention shown in FIG. 1, the length l₃ of the strips 12a and 12b is between about 200 mm and about 205 mm. The absolute lengths of the strips 12a and 12b, however, will vary depending on the overall length of the sanitary napkin 10.

The width of each of the strips 12a and 12b depends upon the values of d₁ and d₂. In the most preferred embodiment described above (when d₁ is about 11 mm and d₂ is about 60 mm) the width of each of the strips is about 24.5 mm. The approximate width of the strips 12a and 12b in other embodiments disclosed herein can be determined by subtracting d₁ from d₂ and dividing the result in half.

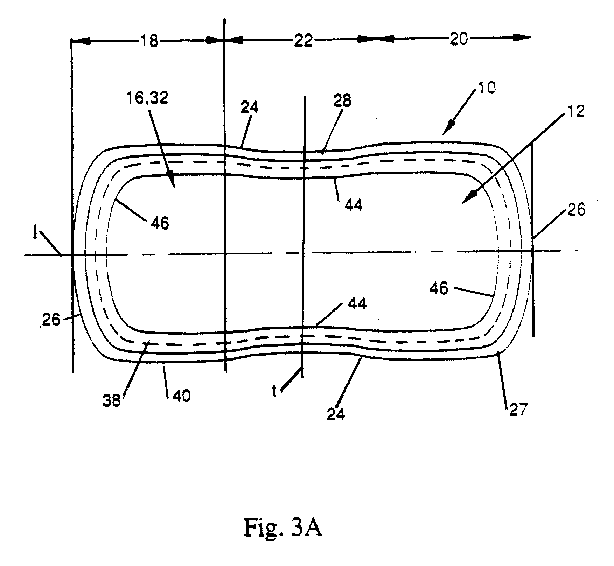

FIG. 3 shows an alternative preferred embodiment of the improved panty fastening adhesive 12 of the present invention. In the alternative embodiment shown in FIG. 3, the distance between the outside edges 44 of the strips and the periphery 28 of the sanitary napkin 10 is approximately the same along the entire periphery 28 of the sanitary napkin 10. Preferably, the distance between the edges 44 and ends 46 of the strips and the periphery 28 of the sanitary napkin 10 is approximately the same throughout the end regions 18 and 20 of the sanitary napkin. As shown in FIG. 3, the distance between the outside edges 44 of the strips and the periphery 28 of the sanitary napkin 10 can also be approximately the same (or uniform) for along the longitudinal edges 24 of the sanitary napkin 10 in the central region 22. In these embodiments based on FIG. 3, the ranges of suitable distances d₁ between the inside edges 42 of the strips are the same as that described for the embodiment shown in FIGS. 1 and 2. The distances d₂ between the outside edges 44 should be greater than about 35 mm in the central region 22 of the sanitary napkin. The outside edges and ends of the strips in the embodiment based on FIG. 3 are preferably at least as close, if not closer to the periphery 28 of the sanitary napkin in the end regions 18 and 20 of the sanitary napkin 10 than they are in the central region 22 of the sanitary napkin 10. The outside edges 44 of the strips should be at least about 6mm, plus or minus 3mm, from the periphery 28 of the sanitary napkin 10 in the central region 22 (that is, from the longitudinal edges). The outside edges 44 can, therefore, be spaced inward toward the longitudinal centerline more than the 6 mm, plus or minus 3 mm spacing provided above. The edges and ends of the strips that are in the end regions 18 and 20 of the sanitary napkin 10, however, should be spaced a distance of about 6 mm, plus or minus 3 mm, from the portion of the periphery 28 which lies in the end regions 18 and 20 of the sanitary napkin 10.

FIG. 3A shows an alternative preferred embodiment of the improved panty fastening adhesive 12 of the present invention shown in FIG. 3. In the alternative embodiment shown in FIG. 3A, the adhesive fastening means 12 comprises a single zone of adhesive rather than two separate strips of adhesive. Because the embodiment shown in FIG. 3A does not provide an area along the longitudinal centerline where the backsheet of the sanitary napkin will be unattached to the wearer's undergarments, it may not have quite the same ability to allow the backsheet to separate from the undergarments as the embodiment shown in FIG. 3. The zone of adhesive 12 can comprise adhesive applied in any pattern so long as the distances from the outside edges 44 and 46 of the zone to the periphery 28 of the sanitary napkin 10 are the same as those specified for the various portions of the periphery 28 of the sanitary napkin for the strips shown in FIG. 3. The spacing between the edges of the zone of adhesive 12 and the periphery 28 of the sanitary napkin 10 (especially in the end regions 18 and 20, and at the corners 27 of the sanitary napkin 10) is particularly important in reducing the edge and end roll over problems described above. The zone of adhesive 12 could be printed on the garment side 32b of the backsheet or applied in any other suitable manner.

FIG. 4 shows another preferred alternative embodiment of the improved panty fastening adhesive 12 of the present invention. In this third embodiment, the sanitary napkin 10 is provided with optional side flaps 39 and the strips 12a and 12b are generally arcuate. In this particular embodiment, the side flaps 39 could be eliminated. In FIG. 4, the strips 12a and 12b are spaced further apart in the end regions of the sanitary napkin than they are in the central region. In other words in the third preferred embodiment, the strips 12a and 12b are inwardly arcuate with relation to each other so that the edges of the strips are closest in the central region 22 of the sanitary napkin 10. As shown in FIG. 4, both the inside edges 42 and the outside edges 44 of the strips 12a and 12b are arcuate. It should be understood, however, that all portions of the arcuate edges of the strips 12a and 12b need not be curvilinear. It is also possible that the longitudinal edges 42 and 44 of the strips 12a and 12b may be comprised of two or more adjacent discrete rectilinear segments, or adjacent rectilinear and curvilinear segments.

In the third embodiment, the distance d₁ between the inside edges 42 of the strips is the same as that described for the embodiments shown in FIGS. 1-3. The distance d₂ between the outside edges 44 of the strips, however, is not necessarily the same for the adhesive fastening means 12 of the third embodiment. The distance d₂ between the outside edges 44 of the strips should be greater than about 35 mm in the central region 22 of the sanitary napkin. The distance between the outside edges 44 in the central region 22 should, however, be less than the distance between the outside edges 44 of the strips in the end regions. Preferably, the distance d₂ between the outside edges 44 of the strips is about 40 mm in the central region 22. In the drawings, the distances between the respective edges of the strips are accompanied by a prime symbol when reference is being made to those distances which are measured at places other than the central region 22 of the sanitary napkin 10. The distance d₂' between the outside edges 44 of the respective strips in the end regions 18 and 20 can range from a distance of greater than about 47 mm to a distance of less than or equal to about 90 mm.

FIG. 5 shows still another preferred alternative embodiment of the improved panty fastening adhesive 12 of the present invention. This fourth embodiment is similar in many respects to the embodiment shown in FIG. 4. However, in the fourth embodiment, the sanitary napkin 10 is asymmetrical about the transverse centerline t. The sanitary napkin 10 shown in FIG. 5 has a second end region 20 which is longer than the first end region 18. This sanitary napkin 10 would generally be worn so the second end region 20 would be to the wearer's rear. Other variations of the sanitary napkin 10 shown in FIG. 5 are possible. For instance, the first end region 18 could be made longer than the second end region 20 and worn in the wearer's front. Alternatively, both ends 26 of the sanitary napkin 10 could be extended, with either the first end region 18 being extended more or less than the second end region 20. In addition, the sanitary napkin 10 shown in FIG. 5 is provided with optional side flaps 39. In this particular embodiment, the side flaps 39 could be eliminated. The approximate configuration the longitudinal edges 24 of the sanitary napkin 10 would take if the side flaps 39 were eliminated is shown with dotted lines in FIG. 5.

The adhesive fastening means 12 of the alternative embodiment shown in FIG. 5 comprises two strips 12a and 12b which are generally arcuate. (It should be noted that one of the strips, 12a, is shown as being intermittent. Strip 12a is shown merely as an example of an intermittent strip for purposes of illustration. Such an intermittent strip or strips could be used in any of the embodiments described herein, and thus this feature is not peculiar to the fourth embodiment.) The configuration of strips 12a and 12b are similar to the strips shown in FIG. 4 in that both sets of strips are generally arcuate. In addition, the strips 12a and 12b are generally inwardly arcuate in the central region 22 of the sanitary napkin. The strips 12a and 12b are also spaced further apart in the end regions than in the central region 22. Preferably, the spacing between the strips 12a and 12b shown in FIG. 5 is within the same ranges as those described above for the third embodiment. The strips 12a and 12b are different from those shown in FIG. 4, however, because they are outwardly arcuate with relation to each other in the end regions 18 and 20.

The sanitary napkin 10 having the shape shown in FIG. 5 does not necessarily require strips which are arcuate in different directions in the end regions 18 and 20. Strips of the same general configuration as those shown in any of the other drawing figures could also be used with a sanitary napkin having a shape similar to that shown in FIG. 5. In addition, other variations of the strips 12a and 12b shown in FIG. 5 could be used with the sanitary napkin 10 of FIG. 5. For instance, the strips 12a and 12b could be arcuate in the same direction in both the central region 22 and the first end region 18, and arcuate in a different direction (such as in the direction of the strips shown in FIG. 5) in the second end region 20. Alternatively, the strips 12a and 12b could be arcuate in the same direction in both the central region 22 and the second end region 20, and arcuate in a different direction in the first end region 18. In another alternative, the portions of the strips that extend into the end regions could be rectilinear. In still further alternatives, the strips 12a and 12b may be comprised of two or more adjacent discrete rectilinear segments, or adjacent rectilinear and curvilinear segments. An example of strips that are at least partially comprised of rectiliniear segments is shown in FIG. 5A.

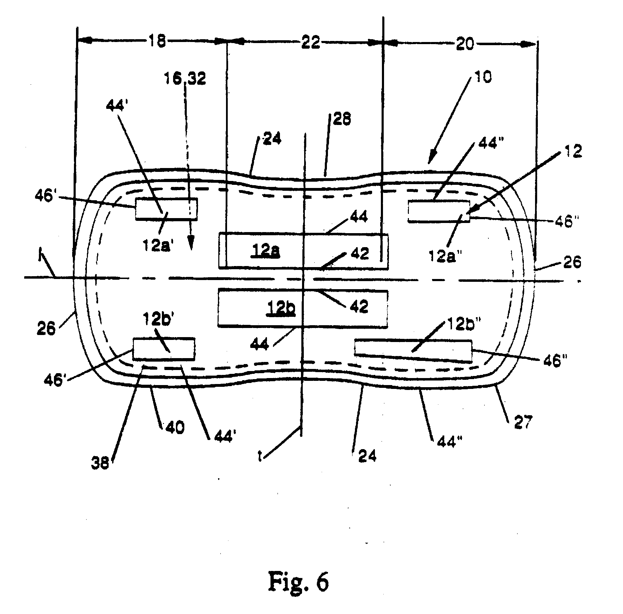

FIG. 6 shows yet another preferred alternative embodiment of the improved adhesive fastening means 12 of the present invention. The adhesive fastening means 12 of the alternative embodiment shown in FIG. 6 comprises two spaced apart longitudinally-oriented zones of adhesive, such as strips 12a and 12b, which are disposed on opposite sides of the longitudinal centerline l of the sanitary napkin 10. The strips 12a and 12b extend at least the length of the central region 22 of the sanitary napkin 10. The strips 12a and 12b shown in FIG. 6 are generally rectangular and parallel to the longitudinal centerline and thus are similar to the strips shown in FIG. 1. The strips 12a and 12b could, however, take the form of any of the zones or strips shown in the other drawing figures, or that are otherwise disclosed herein. The spacing between the strips 12a and 12b shown in FIG. 6 is the same as that specified for the strips shown in FIGS. 1 and 2. If the strips take the form of any of the other zones or strips disclosed herein, such as the arcuate strips shown in FIG. 3, the spacing between the strips should be the same as that specified for the respective (in this case, arcuate) embodiment.

The adhesive fastening means 12 of the alternative embodiment shown in FIG. 6 further comprises at least four additional corner zones of adhesive, 12a', 12a'', 12b', and 12b'' that are each disposed adjacent one corner 27 of the sanitary napkin 10. There is one corner zone in each end region of the sanitary napkin on each side of the longitudinal centerline. Although the corner zones are shown in FIG. 6 as being rectangular (or at least in the shape of polygons), they can be of any shape. Preferably, the edges of the corner zones which are nearest the corners 27 of the sanitary napkin have roughly the same shape as the corners 27. As shown in FIG. 6, the corner zones 12a', 12a'', 12b', and 12b'' are generally positioned further from the longitudinal centerline than the longitudinal strips 12a and 12b. The corner zones and the longitudinal strips, therefore, are generally nonlinear. Preferably, the outside edges of the corner zones 44' and 44'', and the ends of the corner zones which are nearest the periphery 28 of the sanitary napkin 46' and 46'', are spaced inward from the periphery 28 a distance of about 6 mm, plus or minus 3 mm. Preferably, for the embodiment shown in FIG. 6, the absolute distance from one outside edge 44 of one corner zone to the outside edge of the other corner zone should be greater than about 47 mm and less than or equal to about 90 mm.

While particular embodiments of the present invention have been illustrated and described, it would be obvious to those skilled in the art that various other changes and modifications can be made. It is therefore intended to cover in the appended Claims all such changes and modifications that are within the scope of this invention.

## Claims

1. An absorbent article (10) having a caliper of less than or equal to 5 millimetres, a central region (22) disposed between two end regions (18,20) a periphery (28), a portion of said periphery bounding said central region (22), and a portion of said periphery bounding said end regions (18,20), said absorbent article (10) comprising a liquid pervious topsheet (30); a liquid impervious backsheet (32), said backsheet (32) having a core-facing side and a garment side; an absorbent core (34) positioned between said topsheet (30) and said backsheet (32); and an adhesive fastening means (12) on said garment side of said backsheet (32) for attaching said absorbent article (10) to a garment, characterized in that
said fastening means (12) comprises at least one zone of adhesive having outside edges and ends spaced a distance of 6 millimetres, plus or minus 3 millimetres, from said portion of said periphery (28) which bounds the end regions (18,20) of said absorbent article (10).

2. An absorbent article (10)according to claim 1 characterized in that said article (10) has a longitudinal centerline (l), a transverse centerline (t), two spaced apart longitudinal edges (24), two spaced apart end edges (26), said longitudinal and end edges (24,26) of said absorbent article (10) being arcuate, wherein said longitudinal edges (24) are inwardly arcuate with relation to each other in said central region (22) and convex outwardly in said end regions (18,20), and said end edges (24,26) are convex outwardly, said fastening means (12) comprising two spaced apart longitudinally-oriented strips (12a,12b) of adhesive disposed on opposite sides of said longitudinal centerline (l), said strips of adhesive (12a,12b)
each have a longitudinal dimension, a transverse dimension, ends (46), an inside longitudinal edge (42) and an outside longitudinal edge (44) that is disposed further away from the longitudinal centerline (l) of said absorbent article (10) than said inside edge (42), wherein the distance between said inside edges (42) is between 8 millimetres and 30 millimetres in said central region (22) and thedistance between said outside edges (44) is greater than 35 millimetres in said central region (22).

3. An absorbent article (10) according to claim 2 characterized in that said two spaced apart longitudinally-oriented strips (12a,12b) of adhesive are inwardly arcuate and the distance between said outside edges (44) of said strips (12a,12b) of adhesive is greater than 47 millimetres and less than or equal to 90 millimetres in said end regions (18,20).

4. An absorbent article (10) according to claim 2 or 3 characterized in that said
two strips of adhesive (12a,12b)extend at least the length of the central region (22) of the absorbent article (10), and the distance between said outside edges (44) of said strips of adhesive is greater than 47 millimetres in said central region (22), and said fastening means (12) further comprises
four corner zones of adhesive (12a',12a'',12b',12b'') that are each positioned adjacent one corner (27) of said absorbent article, said corner zones (12a',12a'',12b',12b'') having outside edges which are positioned further from the longitudinal centerline (l) than the outside edges of the longitudinal strips, and said outside edges of said corner zones, and the ends of said corner zones which are nearest the periphery of the sanitary napkin are spaced inward from the periphery a distance of 6 mm, plus or minus 3 mm.

5. An absorbent article (10) according to claim 2 characterized in that the distance between said outside edges (44) of said strips of adhesive (12a,12b) is greater than 47 millimetres in said central region (22), and
the inwardly arcuate segments forming the longitudinal edges (24) of said absorbent article (10) in said central region (22) have a center on the transverse centerline and radii of curvature of 100 - 300 mm, more preferably 127 mm,
the convex outwardly segments which form the longitudinal edges (24) of said absorbent article (10) in the end regions (26) have a center located 94 mm from the longitudinal centerline (l) and 73 mm from the transverse centerline (t) and a radii of curvature of up to 500 mm, more preferably 146 mm,
the convex outwardly segments that form the end regions (26) of the absorbent article (10) have a radii of curvature of between 50 - 200 mm, more preferably 113 mm, and the radii of curvature of the corners of the absorbent article (10) is between 10 - 30 mm, more preferably 17 mm.

## Patentansprüche

1. Ein absorbierender Artikel (10) mit einer Abgreifhöhe von weniger als oder gleich wie 5 mm, einem zwischen zwei Endbereichen (18, 20) angeordneten mittleren Bereich (22), einer Peripherie (28), wobei ein Abschnitt der genannten Peripherie den genannten mittleren Bereich (22) begrenzt und ein Abschnitt der genannten Peripherie die genannten Endbereiche (18, 20) begrenzt, wobei der genannte absorbierende Artikel (10) ein flüssigkeitsdurchlässiges Deckblatt (30); ein flüssigkeitsundurchlässiges Rückenblatt (32), wobei das genannte Rückenblatt (32) eine dem Kern zugewandte Seite und eine dem Kleidungsstück zugewandte Seite aufweist; einen absorbierenden Kern (34), welcher zwischen dem genannten Deckblatt (30) und dem genannten Rückenblatt (32) positioniert ist; und ein haftendes Befestigungsmittel (12) an der genannten Kleidungsstückseite des genannten Rückenblattes (32) aufweist, um den genannten absorbierenden Artikel (10) an einem Kleidungsstück zu befestigen, dadurch gekennzeichnet, daß das genannte Befestigungsmittel (12) mindestens eine Haftmittelzone mit außenseitigen Rändern und Enden aufweist, welche in einer Distanz von 6 mm, plus oder minus 3 mm, vom genannten Abschnitt der genannten Peripherie (28) beabstandet sind, welcher die Endbereiche (18, 20) des genannten absorbierenden Artikels (10) begrenzt.

2. Ein absorbierender Artikel (10) nach Anspruch 1, dadurch gekennzeichnet, daß der genannte Artikel (10) eine Längsmittellinie (l), eine Quermittellinie (t), zwei voneinander beabstandete Längsränder (24), zwei voneinander beabstandete Endränder (26) aufweist, wobei die genannten Längs- und Endränder (24, 26) des genannten absorbierenden Artikels (10) gebogen sind, bei welchem die genannten Längsränder (24) im genannten mittleren Bereich (22) in bezug aufeinander einwärts gebogen und in den genannten Endbereichen (18, 20) konvex auswärts sind, und die genannten Endränder (26) konvex auswärts sind, wobei das genannte Befestigungsmittel (12) zwei voneinander beabstandete der Länge nach ausgerichtete Haftstreifen (12a, 12b) umfaßt, welche an gegenüberliegenden Seiten der genannten Längsmittellinie (l) angeordnet sind, wobei die genannten Haftstreifen (12a, 12b) jeweils eine Längsdimension, eine Querdimension, Enden (46), einen inneren Längsrand (42) und einen äußeren Längsrand (44), welcher von der Längsmittellinie (l) des genannten absorbierenden Artikels (10) weiter weg angeordnet ist als der genannte innere Rand (42), aufweist, bei welchem im genannten mittleren Bereich (22) die Distanz zwischen den genannten inneren Rändern (42) zwischen 8 mm und 30 mm ist und die Distanz zwischen den genannten äußeren Rändern (44) im genannten mittleren Bereich (22) größer als 35 mm ist.

3. Ein absorbierender Artikel (10) nach Anspruch 2, dadurch gekennzeichnet, daß die genannten zwei voneinander beabstandeten der Länge nach ausgerichteten Haftstreifen (12a, 12b) nach innen gebogen sind und die Distanz zwischen den genannten äußeren Rändern (44) der genannten Haftstreifen (12a, 12b) größer als 47 mm und weniger als oder gleich wie 90 mm in den genannten Endbereichen (18, 20) ist.

4. Ein absorbierender Artikel (10) nach Anspruch 2 oder 3, dadurch gekennzeichnet, daß die genannten zwei Haftstreifen (12a, 12b) sich über mindestens die Länge des mittleren Bereiches (22) des absorbierenden Artikels (10) erstrecken und die Distanz zwischen den genannten äußeren Rändern (44) der genannten Haftstreifen im genannten mittleren Bereich (22) größer als 47 mm ist und daß das genannte Befestigungsmittel (12) weiters vier Eckzonen von Haftmittel (12a', 12a'', 12b', 12b'') aufweist, welche jeweils nahe einer Ecke (27) des genannnten absorbierenden Artikels positioniert sind, wobei die genannten Eckzonen (12a', 12a'', 12b', 12'') Außenränder aufweisen, welche von der Längsmittellinie (l) weiter positioniert sind als die äußeren Ränder der Längsstreifen, und daß die genannten äußeren Ränder der genannten Eckzonen und die Enden der genannten Eckzonen, welche am nächsten zur Peripherie der Damenbinde sind, von der Peripherie über eine Distanz von 6 mm, plus oder minus 3 mm, einwärts beabstandet sind.

5. Ein absorbierender Artikel (10) nach Anspruch 2, dadurch gekennzeichnet, daß die Distanz zwischen den genannten äußeren Rändern (44) der genannten Haftstreifen (12a, 12b) im genannten mittleren Bereich (22) größer als 47 mm ist und
daß die genannten einwärts gebogenen Segmente, welche die Längsränder (24) des genannten absorbierenden Artikels (10) im genannten mittleren Bereich (22) bilden, eine Mitte an der Quermittellinie und Krümmungsradien von 100 bis 300 mm, bevorzugter 127 mm, aufweisen,
die auswärts konvexen Segmente, welche die Längsränder (24) des genannten absorbierenden Artikels (10) in den Endbereichen (26) bilden, eine Mitte, welche 94 mm von der Längsmittellinie (l) und 73 mm von der Quermittellinie (t) angeordnet ist, und einen Krümmungsradius von bis zu 500 mm, bevorzugter 146 mm, aufweisen,
wobei die auswärts konvexen Segmente, welche die Endbereiche (26) des absorbierenden Artikels (10) bilden, einen Krümmungsradius von zwischen 50 bis 200 mm, bevorzugter 113 mm, aufweisen und der Krümmungsradius der Ecken des absorbierenden Artikels (10) zwischen 10 bis 30 mm, bevorzugter 17 mm, ist.

## Revendications

1. Un article absorbant (10) ayant une épaisseur inférieure ou égale à 5 millimètres, une partie centrale (22) disposée entre deux parties d'extrémité (18, 20), une périphérie (28), une partie de ladite périphérie entourant ladite partie centrale (22), et une partie de ladite périphérie entourant lesdites parties d'extrémité (18, 20), ledit article absorbant (10) comprenant une feuille de dessus perméable aux liquides (30), une feuille de fond imperméable aux liquides (32), ladite feuille de fond (32) ayant une face située en regard de l'âme et une face située en regard du vêtement; une âme absorbante (34) disposée entre ladite feuille de dessus (30) et ladite feuille de fond (32); et un moyen de fixation adhésive (12) sur ladite face située en regard du vêtement de ladite feuille de fond (32) pour fixer ledit article absorbant (10) à un vêtement, caractérisé en ce que:
ledit moyen de fixation (12) comprend au moins une zone d'adhésif ayant des bords extérieurs et des extrémités espacés d'une distance de 6 millimètres, plus ou moins 3 millimètres, de ladite partie de ladite périphérie (28) qui entoure les parties d'extrémité (18, 20) dudit article absorbant (10).

2. Un article absorbant (10) selon la revendication 1, caractérisé en ce que ledit article (10) a une ligne médiane longitudinale (l), une ligne médiane transversale (t), deux bords longitudinaux espacés (24), deux bords d'extrémité espacés (26), lesdits bords longitudinaux et d'extrémité (24, 26) dudit article absorbant (10) étant arqués, et dans lequel lesdits bords longitudinaux (24) sont arqués vers l'intérieur l'un par rapport à l'autre dans ladite partie centrale (22) et convexes vers l'extérieur dans lesdites parties d'extrémité (18, 20), et lesdits bords d'extrémité (24, 26) sont convexes vers l'extérieur, ledit moyen de fixation (12) comprenant deux bandes espacées orientées longitudinalement (12a, 12b) d'adhésif disposées sur les côtés opposés de ladite ligne médiane longitudinale (l), lesdits bandes d'adhésif (12a, 12b) ont chacune une dimension longitudinale, une dimension transversale, des extrémités (46), un bord longitudinal intérieur (42) et un bord longitudinal extérieur (44) qui est disposé en étant éloigné davantage de la ligne médiane longitudinale (l) dudit article absorbant (10) que ledit bord intérieur (42), dans lequel la distance entre lesdits bords intérieurs (42) va de 8 millimètres à 30 millimètres dans ladite partie centrale (22) et la distance entre lesdits bords extérieurs (44) est supérieure à 35 millimètres dans ladite partie centrale (22).

3. Un article absorbant (10) selon la revendication 2, caractérisé en ce que les deux dites bandes espacées, orientées longitudinalement (12a, 12b) d'adhésif sont arquées vers l'intérieur et la distance entre lesdits bords extérieurs (44) desdites bandes (12a, 12b) de l'adhésif est plus grande que 47 millimètres et inférieure ou égale à 90 millimètres dans lesdites parties d'extrémité (18, 20).

4. Un article absorbant (10), selon la revendication 2 ou 3, caractérisé en ce que les deux dites bandes d'adhésif (12a, 12b) s'étendent au moins sur la longueur de ladite partie centrale (22) de l'article absorbant (10), et la distance entre lesdits bords extérieurs (44) desdites bandes d'adhésif est plus grande que 47 millimètres dans ladite partie centrale (22), et ledit moyen de fixation (12) comporte, en outre, quatre zones de coin d'adhésif (12a', 12a'', 12b', 12b'') qui sont disposées près de chaque coin (27) dudit article absorbant, lesdites zones de coin (12a', 12a'', 12b', 12b'') ayant des bords extérieurs qui sont disposés en étant éloignés davantage de la ligne médiane longitudinale (l) que les bords extérieurs des bandes longitudinales, et lesdits bords extérieurs desdites zones de coin et les extrémités desdites zones de coin qui sont les plus proches de la périphérie de la serviette hygiénique sont espacés vers l'intérieur, à partir de la périphérie, d'une distance de 6 mm, plus ou moins 3 mm.

5. Un article absorbant (10) selon la revendication 2, caractérisé en ce que la distance entre lesdits bords extérieurs (44) desdites bandes d'adhésif (12a, 12b) est supérieure à 47 millimètres dans ladite partie centrale (22), et
les segments arqués vers l'intérieur formant les bords longitudinaux (24) dudit article absorbant (10) dans ladite partie centrale (22) ont un centre sur la ligne médiane transversale et un rayon de courbure de 100-300 mm, et de préférence de 127 mm,
les segments convexes vers l'extérieur qui forment les bords longitudinaux (24) dudit article absorbant (10) dans les parties d'extrémité (26) ont un centre disposé à 94 mm de la ligne médiane longitudinale (l) et 73 mm de la ligne médiane transversale (t) et un rayon de courbure allant jusqu'à 500 mm, et de préférence de 146 mm,
les segments convexes vers l'extérieur qui forment les parties d'extrémité (26) de l'article absorbant (10) ont un rayon de courbure entre 50-200 mm, et, de préférence, de 113 mm, et le rayon de courbure des coins de l'article absorbant (10) est entre 10-30 mm, et, de préférence, de 17 mm.
